# EUROPÄISCHE PATENTANMELDUNG

(11) **EP 4 032 564 A1**
(43) Veröffentlichungstag der Anmeldung: **27.07.2022**
(21) Anmeldenummer: 22152051.3
(22) Anmeldetag: 18.01.2022
(51) Int. Cl.: A61M 1/00

(54) **OPHTHALMOLOGISCHE KASSETTE, OPHTHALMOLOGISCHE KONSOLE SOWIE SYSTEM AUS OPHTHALMOLOGISCHER KASSETTE UND KONSOLE**

(30) Priorität: 20.01.2021 DE 102021200471; 04.02.2021 DE 102021201040
(71) Anmelder: Geuder AG, 69126 Heidelberg (DE)
(72) Erfinder: Draheim, René, 69207 Sandhausen (DE); Karschny, Sebastian, 68199 Mannheim (DE); Prisching, Thomas, 68775 Ketsch (DE); Pelka, Falko, 75050 Gemmingen (DE)
(74) Vertreter: Ullrich & Naumann PartG mbB

(57) **Zusammenfassung**

Eine Ophthalmologische Kassette mit einem Gehäuse (1) und mindestens einem flexiblen Dichtelement (8), wobei innerhalb des Gehäuses (1) mindestens ein Fluidkanal (5) zur Förderung eines Fluidums ausgebildet ist, wobei der Fluidkanal (5) ein Aufnahmebecken (11) aufweist, in das Fluidum über einen Fluideingang (12) einströmt und über einen Fluidausgang (13) ausströmt, wobei zumindest ein Teil des flexiblen Dichtelements (5) in den Fluideingang (12) eindrückbar ist, um diesen zu verschließen und wobei zwischen dem Fluideingang (12) und dem Aufnahmebecken (11) mindestens ein Steg (14) als Auflager für das Dichtelement (8) ausgebildet ist, um das Dichtelement (8) gegen ein selbststätiges Verschließen des Fluideingangs (12) zu hemmen. Des Weiteren sind eine ophthalmologische Konsole sowie ein System mit einer ophthalmologischen Konsole und einer ophthalmologischen Kassette beschrieben.

## Beschreibung

Die Erfindung betrifft eine ophthalmologische Kassette mit einem Gehäuse und mindestens einem flexiblen Dichtelement.

Des Weiteren betrifft die Erfindung eine ophthalmologische Konsole sowie ein ophthalmologisches System.

Bei ophthalmologischen Eingriffen besteht oftmals die Notwendigkeit dem Auge Flüssigkeit zuzuführen (sog. Irrigation) sowie Flüssigkeit aus dem Auge abzuführen bzw. abzusaugen (sog. Aspiration). Die Irrigationsflüssigkeit und die Aspirationsflüssigkeit werden hierbei regelmäßig über eine Konsole zur Verfügung gestellt, die mit den entsprechenden chirurgischen Handgeräten beispielsweise über eine Schlauchverbindung verbunden ist. Beispielsweise wird bei einer Phakoemulsifikation dem Auge Irrigationsflüssigkeit zugeführt während die getrübte Linse mittels Ultraschall zerkleinert und die Linsentrümmer zusammen mit der Irrigationsflüssigkeit aus dem Auge abgesaugt werden. Um ein steriles Arbeiten zu ermöglichen, wird die Flüssigkeit über eine ophthalmologische Kassette geführt, die in die Konsole eingebringbar ist, wobei die Kassette nach jedem Eingriff entsorgt wird.

Während des Eingriffes am Auge ist es notwendig, die Irrigation und die Aspiration derart zu steuern. Daher ist es aus der Praxis bekannt, Ventile in der Kassette vorzusehen, welche eine Unterbrechung bzw. Freigabe des Fluidkanals ermöglichen, um den Fluss des Fluidums innerhalb der Kassette zu beeinflussen.

Beispielsweise ist aus dem Dokument EP 3 318 290 B1 eine ophthalmologische Kassette mit einem Ventil vorbekannt. Dabei weist der Fluidkanal der Kassette eine Seitenwandung auf, die aus einem Weichkunststoff gebildet ist, der durch einen Stößel der Konsole eingedrückt werden kann, um den Fluidkanal zu verschließen. Da das Fluidum in einer ophthalmologischen Kassette durch Unterdruck gefördert wird, besteht bei der bekannten Konstruktion das Problem, dass der Weichkunststoff angesaugt wird, was zu einem selbststätigen Verschließen des Ventils führt. Aufgrund der immer kleiner werdenden Instrumente und sich weiter entwickelnder Operationstechniken besteht ein Bedürfnis hin zu höheren Unterdrücken in den Fluidkanälen, was die Problematik des selbsttätigen Verschließens weiter verstärkt. Der vorliegenden Erfindung liegt daher die Aufgabe zugrunde, eine ophthalmologische Kassette der eingangs genannten Art derart auszugestalten und weiterzubilden, dass auch bei höherem Unterdruck mit konstruktiv einfachen Mitteln der Fluss des Fluidums zuverlässig anpassbar ist. Des Weiteren sollen eine verbesserte ophthalmologische Konsole und ein verbessertes ophthalmologisches System angegeben werden.

Erfindungsgemäß wird die voranstehende Aufgabe durch die Merkmale des Anspruches 1 gelöst. Damit ist eine ophthalmologische Kassette mit einem Gehäuse und mindestens einem flexiblen Dichtelement angegeben, wobei innerhalb des Gehäuses mindestens ein Fluidkanal zur Förderung eines Fluidums ausgebildet ist, wobei der Fluidkanal ein Aufnahmebecken aufweist, in das Fluidum über einen Fluideingang einströmt und über einen Fluidausgang ausströmt, wobei zumindest ein Teil des flexiblen Dichtelements in den Fluideingang eindrückbar ist, um diesen zu verschließen und wobei zwischen dem Fluideingang und dem Aufnahmebecken mindestens ein Steg als Auflager für das Dichtelement ausgebildet ist, um das Dichtelement gegen ein selbsttätiges Verschließen des Fluideingangs zu hemmen.

In erfindungsgemäßer Weise ist zunächst erkannt worden, dass die zugrundeliegende Aufgabe in verblüffend einfacher Weise gelöst werden kann, indem ein Aufnahmebecken angeordnet ist, in das Fluidum über einen Fluideingang einströmen kann, wobei zwischen dem Fluideingang und dem Aufnahmebecken mindestens ein Steg angeordnet ist. Der Steg dient dabei als Auflager für das Dichtelement, so dass verhindert wird, dass das flexible Dichtelement durch einen in dem Fluidkanal herrschenden Unterdruck angesaugt wird. Somit wird ein selbsttätiges Verschließen des Fluideingangs durch das Dichtelement verhindert. Mit anderen Worten ist durch die Kombination des Dichtelements, des Fluideingangs, des Aufnahmebeckens, des Fluideingangs, des Fluidausgangs und des mindestens einen Stegs ein Ventil geschaffen, das beispielsweise durch einen konsolenseitig angeordneten Stempel geschlossen werden kann. Dadurch wird das flexible Dichtelement in den Fluideingang eingedrückt. Um das Ventil zu öffnen wird der Stempel zurückgefahren, so dass das Dichtelement aufgrund seiner Flexibilität aus dem Fluideingang in seine Ursprungslage zurückkehrt. Dabei wird ein selbsttätiges Schließen des Ventils stets verhindert.

Durch die erfindungsgemäße Konstruktion kann es gemäß einer bevorzugten Ausgestaltung ermöglicht werden, chirurgische Instrumente mit extrem kleinen Durchmessern von 25 Gauge bis hin zu 30 Gauge mit Fluidum zu versorgen bzw. Fluidum durch diese zu aspirieren, wozu beispielsweise ein Unterdruck im Bereich von ca. 0,9 bar notwendig ist. In vorteilhafter Weise können mindestens zwei Fluidkanäle ausgebildet sein, ein Aspirationskanal und ein Irrigationskanal, die jeweils eine erfindungsgemäße Ventilkonstruktion mit einem Aufnahmebecken, einem Fluideingang, einem Fluidausgang, einem flexiblen Dichtelement und einem Steg als Auflager aufweisen. Alternativ oder zusätzlich können mehrere Fluidkanäle zur Irrigation und/oder Aspiration ausgebildet sein, wobei die Aspirationskanäle bzw. die Irrigationskanäle jeweils miteinander über eine erfindungsgemäße Ventilkonstruktion verbunden sind. Durch die Betätigung der Ventilkonstruktionen ist es somit möglich, den Fluss des Fluidums durch den gewünschten Zweig des Aspirationskanals bzw. des Irrigationskanals zu lenken. Unabhängig von der Anzahl der Fluidkanäle ist es denkbar, dass der Fluidkanal bzw. die Fluidkanäle auf zwei Ebenen innerhalb des Gehäuses verlaufen.

In weiter vorteilhafter Weise können drei Stege, vorzugsweise vier Stege, angeordnet sein. Eine solche Konstruktion hat den Vorteil, dass das Dichtelement noch besser gegen ein selbsttätiges Verschließen gesichert ist und eine ausreichend große Strömungsverbindung realisiert ist, um Fluidum zu fördern.

In besonders vorteilhafter Weise kann der Fluidausgang durch eine Ausnehmung in einer Seitenwandung des Aufnahmebeckens gebildet sein. Die Höhe der Seitenwandung kann zumindest im Wesentlichen der Höhe des mindestens einen Stegs entsprechen und somit ebenfalls als Auflager für das Dichtelement dienen. Dadurch wird der kombinatorische Effekt erzielt, dass die Seitenwandung den Fluidausgang definiert und gleichzeitig ein selbsttätiges Verschließen der Strömungsverbindung weiter verhindert.

Gemäß einer vorteilhaften Ausgestaltung kann das Aufnahmebecken den Fluideingang zumindest bereichsweise, vorzugsweise vollständig, umschließen. Der Fluideingang könnte somit als Durchgang ausgebildet sein, der von dem Aufnahmebecken umgeben ist, wodurch eine besonders kompakte und gut zu verschließende Ventilkonstruktion realisierbar ist. Alternativ oder zusätzlich könnte das Aufnahmebecken als Ringkanal ausgebildet sein. Der Ringkanal könnte den Fluideingang umgeben, so dass das Fluidum im Wesentlichen radial durch den Steg bzw. die Stege in den Ringkanal strömt. Gemäß einer bevorzugten Ausgestaltung könnte der Fluideingang zumindest im Wesentlichen orthogonal zur Erstreckungsrichtung des Dichtelements verlaufen. In besonders vorteilhafter Weise könnte der Fluideingang als Durchgang ausgebildet sein, der in der Einbaulage der Kassette in der Konsole ermöglicht, dass der Fluidkanal "von unten", d.h. vertikal mit Fluidum befüllbar ist. Dies hat den Vorteil, dass ein Luftleeres befüllen der Kassette mit Fluidum möglich ist, da diese durch die ansteigende Flüssigkeitssäule aus dem Kanal herausgedrückt wird.

In vorteilhafter Weise kann der Fluideingang entgegen oder mit der Strömungsrichtung des Fluidums konisch zulaufend ausgebildet sein. Eine solche Ausgestaltung hat den Vorteil, dass eine besonders fluiddichte Verbindung erreicht werden kann, wenn das Dichtelement in den Fluideingang eingedrückt wird, vorzugsweise durch einen korrespondierend ausgebildeten, konisch zulaufenden Stößel.

Um die Strömung des Fluidums zu verbessern, kann in Strömungsrichtung des Fluidums gesehen eine Seitenwandung des Fluidkanals ausgehend von dem Fluidausgang rampenartig ausgebildet sein.

Gemäß einer vorteilhaften Ausgestaltung kann das Gehäuse aus einem Grundkörper und mindestens einem Gehäusedeckel ausgebildet sein. Eine solche mehrteilige, insbesondere eine zweiteilige Konstruktion hat den Vorteil, dass auf einfache Weise mindestens ein dichter Fluidkanal ausgebildet werden kann. Der Grundkörper und der Gehäusedeckel können dabei aus einem harten Material, insbesondere einem Kunststoff ausgebildet sein. Auch ist es denkbar, dass der Grundköper und der Gehäusedeckel miteinander verschweißt sind, insbesondere im Ultraschallschweißverfahren. Hierzu könnten vorzugsweise in dem Grundkörper E-Schweißgeber ausgebildet sein.

In weiter vorteilhafter Weise kann das Dichtelement zumindest bereichsweise innerhalb des Gehäuses angeordnet sein, vorzugsweise, wobei das Gehäuse im Bereich des Dichtelements mindestens eine Ausnehmung aufweist. Dadurch wird der Vorteil erzielt, dass eine robuste Konstruktion geschaffen ist, wobei das elastische Dichtelement auf einfache Weise konsolenseitig bewegbar ist, um den Fluidkanal zu verschließen.

In besonders vorteilhafter Weise kann das Dichtelement als Teil einer zumindest teilweise innerhalb des Gehäuses angeordneten Membran bzw. Matte ausgebildet sein. Im Konkreten könnte die Membran bereichsweise erhaben ausgebildet sein, um das Dichtelement zu bilden. Alternativ oder zusätzlich kann die Membran aus einem Material mit einer Shore-Härte von mindestens 50 Shore, insbesondere von mindestens 60 Shore, vorzugsweise von 70 Shore hergestellt ist.

Zur Realisierung einer besonders fluiddichten Ausgestaltung der ophthalmologischen Kassette ist es denkbar, dass die Membran zumindest bereichsweise einen Teil der Wandung des Fluidkanals bildet bzw. dichtet. Mit anderen Worten kann somit ein Teil des Fluidkanals bzw. der Fluidkanäle in der Membran ausgebildet sein. Alternativ oder zusätzlich ist es denkbar, dass die Membran Ausnehmungen aufweist, welche ein Verschweißen des Grundkörpers und des Gehäusedeckels ermöglichen.

Gemäß einer vorteilhaften Ausgestaltung ist es möglich, dass mindestens ein Messelement zur Ermittlung des in einem Fluidkanal herrschenden Drucks ausgebildet ist. Das Messelement kann durch einen entsprechend dünn ausgebildeten Messbereich der Membran realisiert sein, an der ein magnetisches Element, beispielsweise eine magnetische Metallscheibe, vorzugsweise eine magnetische Edelstahlscheibe, angeordnet ist, welche an die Konsole ankoppelbar ist. Durch die Auslenkung des magnetischen Elements kann der Druck innerhalb des Fluidkanals bestimmbar sein, beispielsweise über einen konsolenseitig angeordneten Kraftsensor.

In vorteilhafter Weise steht mindestens ein Pulsationsbecken mit dem Fluidkanal in Strömungsverbindung, wobei das Pulsationsbecken zumindest bereichsweise eine flexible Wandung aufweist. Das Pulsationsbecken dient dazu, Pulsationen des Fluidums zu dämpfen bzw. auszugleichen, indem nämlich dessen Wandung zumindest bereichsweise entsprechend flexibel ausgebildet ist, dass diese aufgrund der Pulsationen ausgelenkt wird. Insbesondere Pulsationen in einem zur Irrigation bzw. Infusion dienenden Fluidkanal, die von einer Rollenpumpe hervorgerufen werden, können somit auf einfache Weise gedämpft werden. Die flexible Wandung kann in besonders bevorzugter Weise durch eine Ausdünnung in der Membran gebildet werden, sofern eine solche Membran vorgesehen ist.

In Bezug auf die ophthalmologische Konsole wird die zugrundeliegende Aufgabe durch die Merkmale von Anspruch 14 gelöst. Damit ist eine ophthalmologische Konsole angegeben, mit einem bewegbaren Stempel zum Eindrücken eines Dichtelements einer ophthalmologischen Kassette, insbesondere einer ophthalmologischen Kassette nach einem der Ansprüche 1 bis 13, wobei der Stempel an seinem freien Ende vorzugsweise konisch oder zylindrisch distal abgerundet ausgebildet ist. Durch die konische Ausgestaltung des Stempels wird der Fluidkanal besonders sicher abgedichtet, wenn der Stempel das Dichtelement in den Fluideingang eindrückt. In besonders vorteilhafter Weise ist der Fluideingang ebenfalls konisch ausgebildet, so dass die Geometrie des Stempels und des Dichtelements miteinander korrespondieren.

In Bezug auf das ophthalmologisches System wird die zugrundeliegende Aufgabe des Weiteren durch die Merkmale des Anspruchs 15 gelöst. Damit ist ein ophthalmologisches System angegeben, mit einer ophthalmologischen Kassette nach einem der Ansprüche 1 bis 13 und einer ophthalmologischen Konsole, insbesondere einer ophthalmologischen Konsole nach Anspruch 14.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel einer erfindungsgemäßen ophthalmologischen Kassette,
- Fig. 2: in einer weiteren schematischen Ansicht eine Explosionsdarstellung der ophthalmologischen Kassette gemäß Fig. 1,
- Fig. 3: in einer weiteren schematischen Ansicht eine teilweise geschnittene Darstellung der ophthalmologischen Kassette gemäß Fig. 1,
- Fig. 4: in einer weiteren schematischen Ansicht eine teilweise geschnittene Darstellung der ophthalmologischen Kassette gemäß Fig. 1,
- Fig. 5: in einer schematischen Ansicht einen Teilbereich des Grundkörpers der ophthalmologischen Kassette gemäß Fig. 1,
- Fig. 6: in einer schematischen Ansicht einen Teilbereich der ophthalmologischen Kassette gemäß Fig. 1 sowie eines Teils einer ophthalmologischen Konsole,
- Fig. 7: in einer weiteren schematischen Ansicht einen Teilbereich der ophthalmologischen Kassette gemäß Fig. 1 sowie eines Teils einer ophthalmologischen Konsole, und
- Fig. 8: in einer schematischen Ansicht einen Teilbereich der ophthalmologischen Kassette gemäß Fig. 1 sowie eines Teils einer ophthalmologischen Konsole.

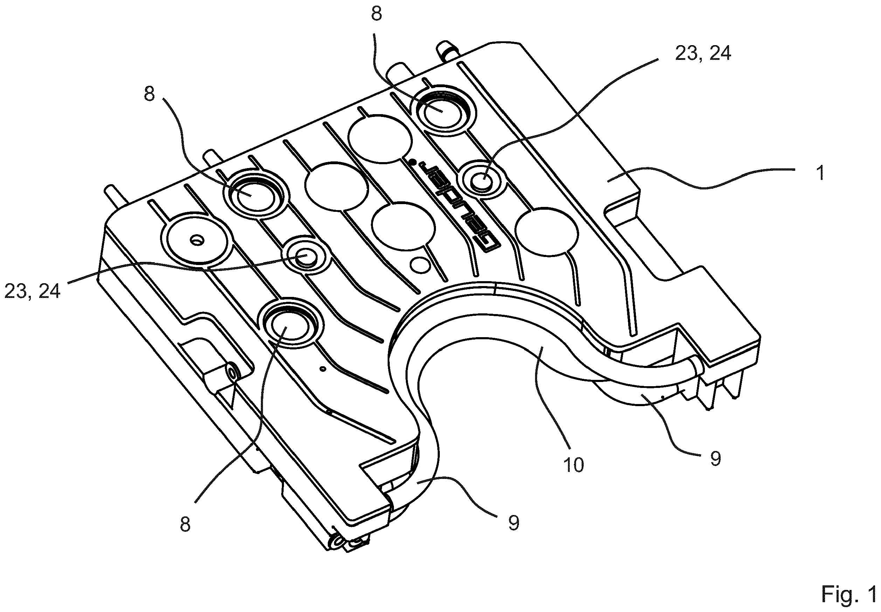

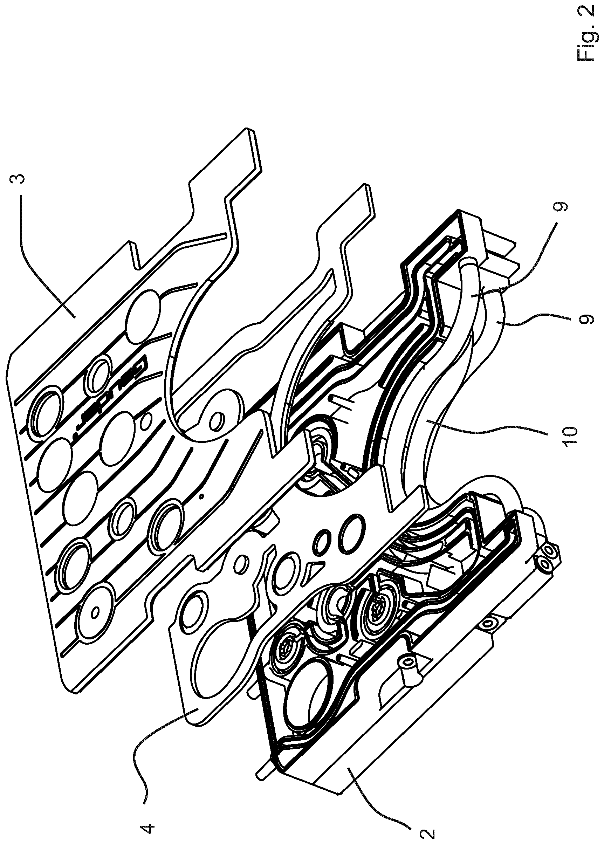

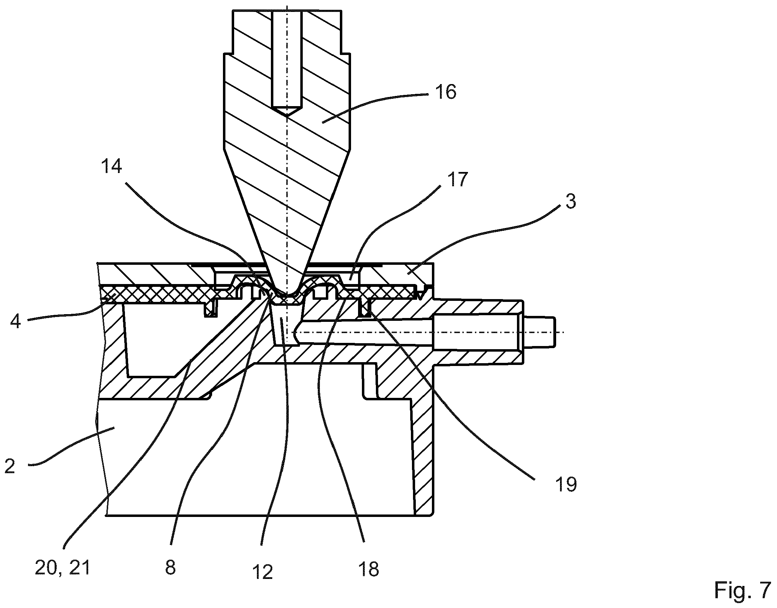

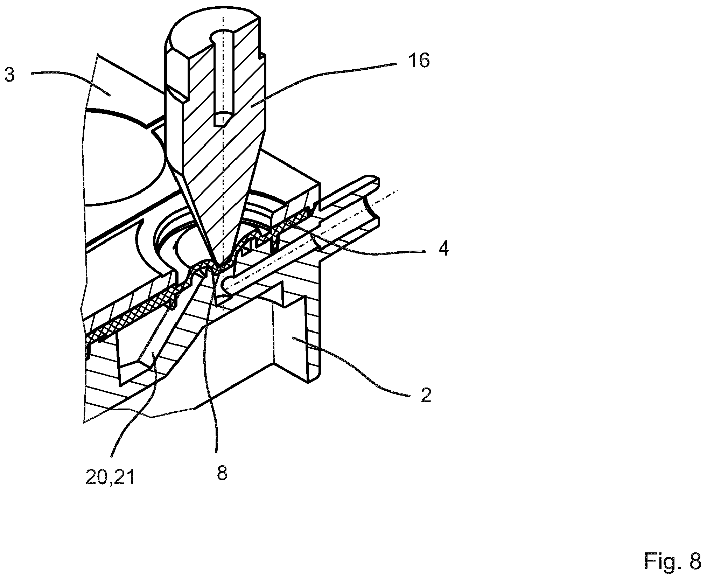

Die Figuren Fig. 1 bis 8 zeigen ein Ausführungsbeispiel einer erfindungsgemäßen ophthalmologischen Kassette, wobei zur Verbesserung der Übersichtlichkeit nicht in jeder Figur jedes Element mit einem Bezugszeichen versehen ist. Die Kassette weist ein Gehäuse 1 auf, das aus einem Grundkörper 2 und einem Gehäusedeckel 3 gebildet ist. Zwischen dem Grundkörper 1 und dem Gehäusedeckel 3 ist eine Membran 4 angeordnet.

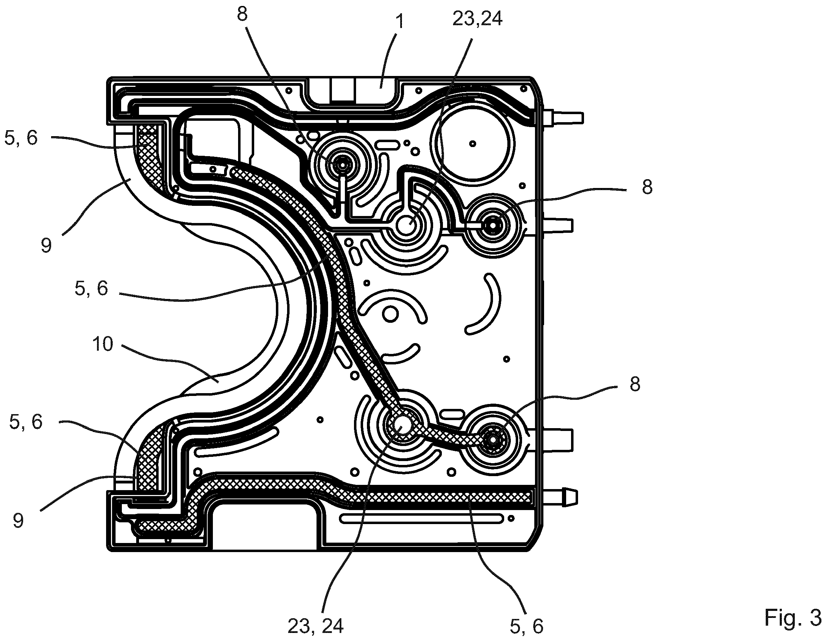

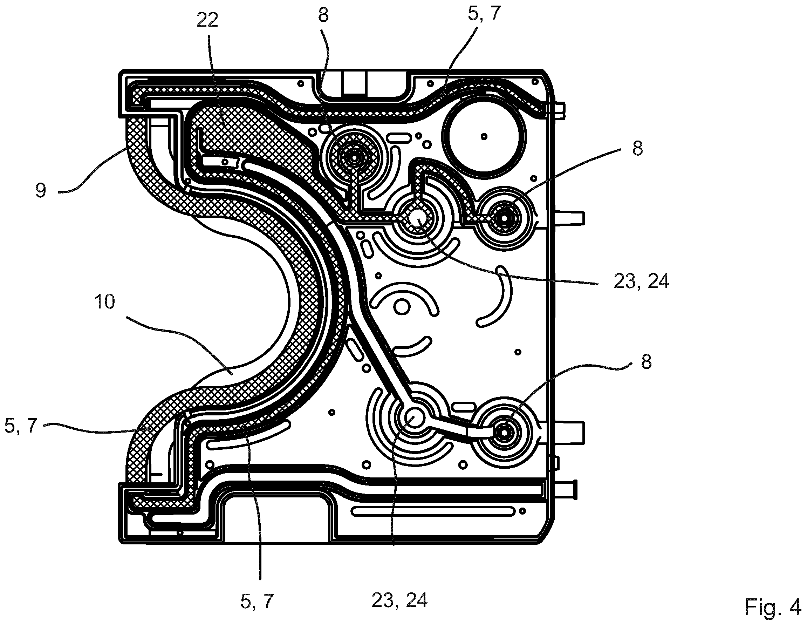

Insbesondere aus Fig. 3 und 4 ist zu erkennen, dass innerhalb des Gehäuses 1 Fluidkanäle 5 verlaufen, wobei die Membran 4 zumindest bereichsweise einen Teil der Wandung der Fluidkanäle 5 bildet. In Fig. 3 ist dabei der Verlauf des Aspirationskanals 6 dargestellt, in Fig. 4 der Verlauf des Irrigationskanals 7. Dabei ist deutlich zu erkennen, dass an dem Aspirationskanal 6 ein Dichtelement 8 und an dem Irrigationskanal zwei Dichtelemente 8 ausgebildet sind, über welche der Aspirationskanal 6 bzw. der Irrigationskanal 7 verschlossen werden können. Des Weiteren weisen der Irrigationskanal 6 und der Aspirationskanal 7 jeweils einen Bereich auf, der durch einen Schlauch 9 realisiert ist. Somit kann eine konsolenseitig angeordnete Rollenpumpe an den Schläuchen 9 angreifen und somit das Fluidum durch den Irrigationskanal 6 bzw. den Aspirationskanal 7 fördern. Die Schläuche 9 sind durch einen am Grundkörper 2 ausgebildeten Mittelsteg 10 voneinander getrennt.

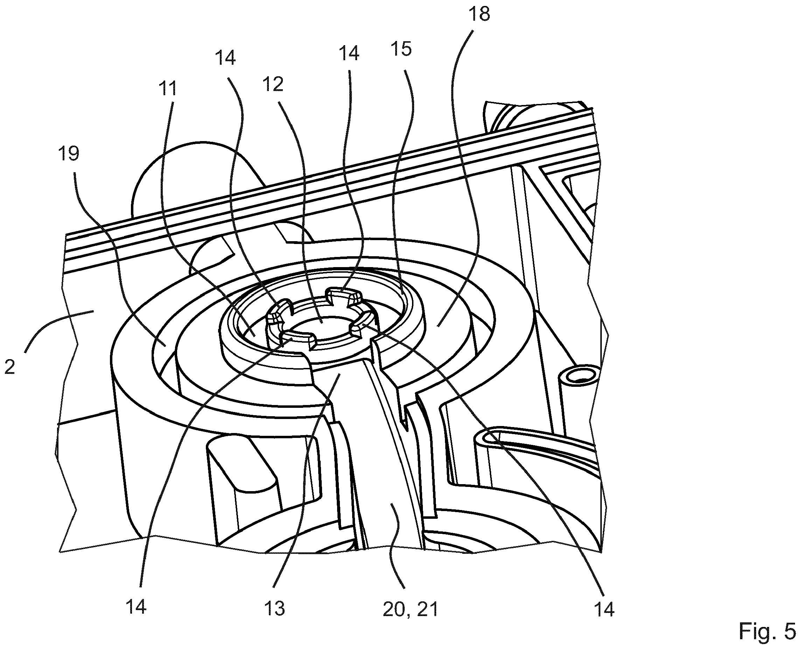

In Fig. 5 ist deutlich zu erkennen, dass im Bereich der Dichtelemente 8 jeweils in dem Fluidkanal 5 ein Aufnahmebecken 11 ausgebildet ist, in das das Fluidum über einen Fluideingang 12 einströmt und über einen Fluidausgang 13 ausströmt. Zwischen dem Fluideingang 12 und dem Fluidausgang 13 sind vier Stege 14 als Auflager für das Dichtelement 8 ausgebildet. Durch die Stege 14 wird verhindert, dass das Dichtelement 8 durch ein in dem Fluidkanal 5 herrschenden Unterdruck in den Fluideingang 12 eingesaugt wird und diesen verschließt, da das Dichtelement 8 auf den Stegen 14 aufliegt und Fluidum durch den Bereich zwischen den Stegen 14 hindurchströmen kann. Ein selbsttätiges Verschließen wird somit verhindert. Es wird dabei darauf hingewiesen, dass auch lediglich ein einziger Steg 14 oder mehr als vier Stege 14 angeordnet sein können.

Das Aufnahmebecken 11 ist in diesem Ausführungsbeispiel als Ringkanal ausgebildet und umschließt den Fluideingang 12, wobei der Fluidausgang 13 durch eine Ausnehmung in der Seitenwandung 15 des Aufnahmebeckens 11 gebildet ist. Die Höhe der Seitenwandung 15 entspricht der Höhe der Stege 14 und dient ebenfalls als Auflager für das Dichtelement 8, wobei dies nicht zwangsweise der Fall sein muss.

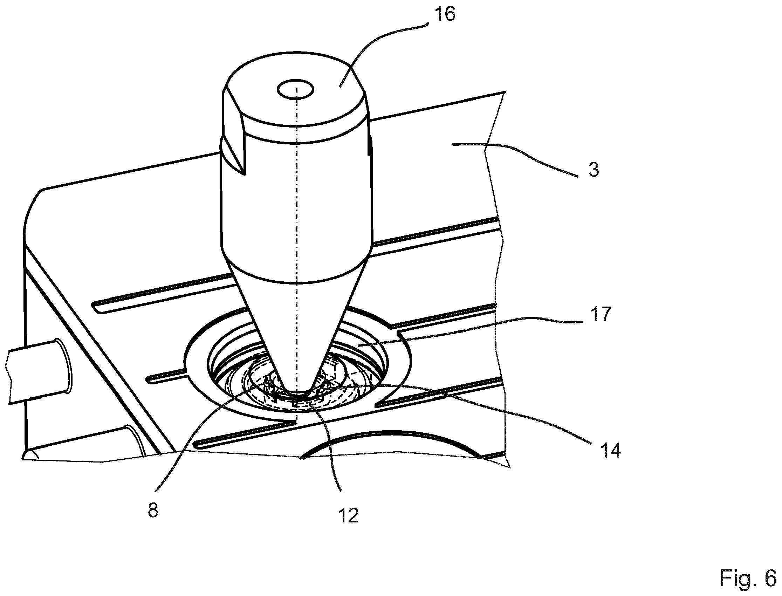

In den Fig. 6 bis 8 ist dargestellt, dass das Dichtelement 8 zum Verschließen des Fluidkanals 5 durch einen konsolenseitig angeordneten Stempel 16 in den Fluideingang 12 eingepresst wird. Hierzu ist in dem Gehäusedeckel 3 im Bereich des Dichtelements 8 eine Ausnehmung 17 vorgesehen. Hierbei sind der Stempel 16 und der Fluideingang 12 jeweils konisch ausgebildet, so dass ein besonders dichtes Verschließen des Fluidkanals 5 erzielt wird, wenn der Stempel 16 in Richtung des Fluideingangs 12 ausgelenkt wird. Nach dem Zurückfahren des Stempels 16 gleicht sich das zuvor gequetschte Dichtelement 8 aufgrund seiner Eigenspannung wieder aus und gibt den Fluideingang 12 frei. Zusätzlich ist eine Auflagefläche 18 ausgebildet, auf der die Membran 4 aufliegt. Um die Auflagefläche 18 herum ist eine Nut 19 vorgesehen, in die eine entsprechend ausgebildete Erhebung an der Membran 4 eingreift, wodurch die Dichtigkeit abermals erhöht wird.

Weiterhin ist zu erkennen, dass eine Seitenwandung 20 des Fluidkanals 5 in Strömungsrichtung des Fluidums gesehen ausgehend von dem Fluidausgang 13 eine Rampe 21 aufweist, d.h. rampenartig ausgebildet ist, wodurch sich die Strömungseigenschaften des Fluidums verbessern.

Um Pulsationen des Fluidums zu dämpfen, die insbesondere durch die Verwendung einer Rollenpumpe hervorgerufen werden, ist gemäß Fig. 4 in dem Irrigationskanal 7 ein Pulsationsbecken 22 ausgebildet. Dieser ist teilweise durch eine Wandung abgeschlossen, die von der Membran 4 realisiert wird und flexibel ausgebildet ist. Hierzu kann die Membran 4 beispielsweise im Bereich des Pulsationsbeckens 22 verdünnt ausgebildet sein.

Zur Bestimmung des Drucks innerhalb der Fluidkanäle 5 sind Messelement 23 angeordnet. Die Messelement 23 sind jeweils durch einen entsprechend dünn ausgebildeten Messbereich der Membran 4 realisiert, an dem ein magnetisches Element 24 beispielsweise eine magnetische Metallscheibe, vorzugsweise eine magnetische Edelstahlscheibe, angeordnet ist, welche an die Konsole ankoppelbar ist. Durch die Auslenkung des magnetischen Elements 24 kann der Druck innerhalb des Fluidkanals 5 bestimmt werden, beispielsweise über einen konsolenseitig angeordneten Kraftsensor.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Gehäuse
- 2: Grundkörper
- 3: Gehäusedeckel
- 4: Membran
- 5: Fluidkanal
- 6: Aspirationskanal
- 7: Irrigationskanal
- 8: Dichtelement
- 9: Schlauch
- 10: Mittelsteg
- 11: Aufnahmebecken
- 12: Fluideingang
- 13: Fluidausgang
- 14: Steg
- 15: Seitenwandung (Aufnahmebecken)
- 16: Stempel
- 17: Ausnehmung
- 18: Auflagefläche
- 19: Nut
- 20: Seitenwandung (Fluidkanal)
- 21: Rampe
- 22: Pulsationsbecken
- 23: Messelement
- 24: magnetisches Element

## Patentansprüche

1. Ophthalmologische Kassette mit einem Gehäuse (1) und mindestens einem flexiblen Dichtelement (8), wobei innerhalb des Gehäuses (1) mindestens ein Fluidkanal (5) zur Förderung eines Fluidums ausgebildet ist, wobei der Fluidkanal (5) ein Aufnahmebecken (11) aufweist, in das Fluidum über einen Fluideingang (12) einströmt und über einen Fluidausgang (13) ausströmt, wobei zumindest ein Teil des flexiblen Dichtelements (5) in den Fluideingang (12) eindrückbar ist, um diesen zu verschließen und wobei zwischen dem Fluideingang (12) und dem Aufnahmebecken (11) mindestens ein Steg (14) als Auflager für das Dichtelement (8) ausgebildet ist, um das Dichtelement (8) gegen ein selbsttätiges Verschließen des Fluideingangs (12) zu hemmen.

2. Ophthalmologische Kassette nach Anspruch 1, **dadurch gekennzeichnet, dass** drei Stege (14), vorzugsweise vier Stege (14), angeordnet sind.

3. Ophthalmologische Kassette nach Anspruch 1 oder 2, **dadurch gekennzeichnet, dass** der Fluidausgang (13) durch eine Ausnehmung in einer Seitenwandung (15) des Aufnahmebeckens (11) gebildet ist.

4. Ophthalmologische Kassette nach einem der Ansprüche 1 bis 3, **dadurch gekennzeichnet, dass** das Aufnahmebecken (11) den Fluideingang (12) zumindest bereichsweise, vorzugsweise vollständig, umschließt.

5. Ophthalmologische Kassette nach einem der Ansprüche 1 bis 4, **dadurch gekennzeichnet, dass** das Aufnahmebecken (11) als Ringkanal ausgebildet ist.

6. Ophthalmologische Kassette nach einem der Ansprüche 1 bis 5, **dadurch gekennzeichnet, dass** der Fluideingang (12) entgegen oder mit der Strömungsrichtung des Fluidums konisch zulaufend ausgebildet ist.

7. Ophthalmologische Kassette nach einem der Ansprüche 1 bis 6, **dadurch gekennzeichnet, dass** der Fluideingang (12) zumindest im Wesentlichen orthogonal zur Erstreckungsrichtung des Dichtelements (8) verläuft.

8. Ophthalmologische Kassette nach einem der Ansprüche 1 bis 7, **dadurch gekennzeichnet, dass** in Strömungsrichtung des Fluidums gesehen eine Seitenwandung (20) des Fluidkanals (5) ausgehend von dem Fluidausgang (13) bevorzugt rampenartig ausgebildet ist.

9. Ophthalmologische Kassette nach einem der Ansprüche 1 bis 8, **dadurch gekennzeichnet, dass** das Gehäuse (1) aus einem Grundkörper (2) und mindestens einem Gehäusedeckel (3) ausgebildet ist.

10. Ophthalmologische Kassette nach einem der Ansprüche 1 bis 9, **dadurch gekennzeichnet, dass** das Dichtelement (8) zumindest bereichsweise innerhalb des Gehäuses (1) angeordnet ist, vorzugsweise, wobei das Gehäuse (1) im Bereich des Dichtelements (8) mindestens eine Ausnehmung (17) aufweist.

11. Ophthalmologische Kassette nach einem der Ansprüche 1 bis 10, **dadurch gekennzeichnet, dass** das Dichtelement (8) als Teil einer zumindest teilweise innerhalb des Gehäuses (1) angeordneten Membran (4) ausgebildet ist.

12. Ophthalmologische Kassette nach Anspruch 11, **dadurch gekennzeichnet, dass** die Membran (4) zumindest bereichsweise einen Teil der Seitenwandung (20) des Fluidkanals (5) bildet bzw. dichtet.

13. Ophthalmologische Kassette nach einem der Ansprüche 1 bis 12, **dadurch gekennzeichnet, dass** mindestens ein Pulsationsbecken (22) mit dem Fluidkanal (5) in Strömungsverbindung steht, wobei das Pulsationsbecken (22) zumindest bereichsweise eine flexible Wandung aufweist, um Pulsationen des Fluidums zu dämpfen bzw. auszugleichen.

14. Ophthalmologische Konsole mit einem bewegbaren Stempel (16) zum Eindrücken eines Dichtelements (8) einer ophthalmologischen Kassette, insbesondere einer ophthalmologischen Kassette nach einem der Ansprüche 1 bis 13, wobei der Stempel (16) an seinem freien Ende vorzugsweise konisch oder zylindrisch distal abgerundet ausgebildet ist.

15. Ophthalmologisches System mit einer ophthalmologischen Kassette nach einem der Ansprüche 1 bis 13 und einer ophthalmologischen Konsole, insbesondere einer ophthalmologischen Konsole nach Anspruch 14.
